# EUROPEAN PATENT APPLICATION

(11) **EP 4 643 787 A1**
(43) Date of publication of application: **05.11.2025**
(21) Application number: 23909897.3
(22) Date of filing: 29.11.2023
(51) Int. Cl.: A61B 17/00, A61B 17/12, A61M 39/06, A61M 25/01

(54) **DELIVERY DEVICE AND DELIVERY SYSTEM**

(30) Priority: 27.12.2022 CN 202211682824
(71) Applicant: Lifetech Scientific (Shenzhen) Co., Ltd., Shenzhen, Guangdong 518063 (CN)
(72) Inventor: WANG, Yu, Shenzhen, Guangdong 518063 (CN); LIU, Jianyong, Shenzhen, Guangdong 518063 (CN)
(74) Representative: Michalski Hüttermann & Partner Patentanwälte mbB
(86) International application number: PCT/CN2023/135060
(87) International publication number: WO 2024/139967

(57) **Abstract**

Provided are a delivery device (100) and a delivery system (200). The delivery device (100) includes: a delivery sheath (30); a hemostasis valve (10), including a valve seat (11), an elastic sealing member (12), and a sheath connector (13), wherein the elastic sealing member (12) is arranged in the valve seat (11), the elastic sealing member (12) has a first state and a second state, a distal end of the sheath connector (13) extends into the valve seat (11) and moves in the valve seat (11) in a axial direction, and the valve seat (11), the elastic sealing member (12), and the sheath connector (13) together define a delivery channel that is communicated with the delivery sheath (30); and an adjusting nut (20), rotatably surrounding the outside of the sheath connector (13), wherein the adjusting nut (20) drives the sheath connector (13) to move in the axial direction. The sheath connector (13) presses the elastic sealing member (12) within a moving path to enable the elastic sealing member to switch between the first state and the second state. When the elastic sealing member is in the first state, the delivery channel is in an open state, and when the elastic sealing member is in the second state, the delivery channel is in a closed state. The hemostasis valve (10) does not need to be disassembled from the delivery device (100) during surgery.

## Description

### Technical Field

The present invention relates to the technical field of interventional medical devices, and particularly, to a delivery device and a delivery system.

### Background Art

Catheter-based interventional methods are commonly adopted for treating cardiovascular diseases. Specifically, various materials and devices are placed into the heart, arteries, veins, and other parts of a human body through catheter intervention to treat cardiovascular diseases.

For example, an interventional medical device, such as an atrial septal defect (ASD) occluder, a ventricular septal defect (VSD) occluder, a patent ductus arteriosus (PDA) occluder, or a patent foramen ovale (PFO) occluder, is delivered to a defect site in the heart by the catheter-based interventional method to block the defect and treat congenital heart diseases.

An existing delivery system includes a loader, a hemostasis valve, a delivery sheath, a delivery cable, and the like. When the abovementioned interventional medical device is delivered to a treatment site in a human body through the delivery system, the hemostasis valve is assembled with the loader or the delivery sheath as needed. Operation steps for using a conventional delivery system during surgery are as follows: after a delivery sheath is inserted into a human body, a hemostasis valve is connected to a tail portion of the delivery sheath for hemostasis; another hemostasis valve is then assembled with a loader; after assembly, a medical device is accommodated inside the loader, and air evacuation is performed; the hemostasis valve connected to the tail portion of the delivery sheath is removed, and a head of the loader is inserted into the tail portion of the delivery sheath; and a connecting thread is tightened, and a delivery cable is used to push the medical device from the loader into the delivery sheath to complete the implantation in the body. The existing delivery system increases surgical steps and extends operation time due to the repeated assembly and disassembly of the hemostasis valve and the delivery sheath in an interventional process. Additionally, there is a risk of the loader detaching or loosening after being assembled with the delivery sheath, which may result in leakage.

### Summary of the Invention

In order to overcome the above-mentioned drawbacks, the present invention aims to at least provide a delivery device and delivery system that do not require the disassembly of a hemostasis valve during surgery.

The objective is achieved through the following technical solutions.

According to a technical solution in a first aspect of the present invention, a delivery device is proposed.

The delivery device includes:
a delivery sheath;
a hemostasis valve, which includes a valve seat, an elastic sealing member, and a sheath connector, wherein the elastic sealing member is arranged in the valve seat, the elastic sealing member has a first state and a second state, a distal end of the sheath connector extends into the valve seat and moves in the valve seat in an axial direction, and the valve seat, the elastic sealing member, and the sheath connector together define a delivery channel that is communicated with the delivery sheath; and
an adjusting nut, rotatably surrounding the outside of the sheath connector, wherein the adjusting nut drives the sheath connector to move in the axial direction,
wherein the sheath connector presses the elastic sealing member within a moving path to enable the elastic sealing member to switch between the first state and the second state, when the elastic sealing member is in the first state, the delivery channel is in an open state, and when the elastic sealing member is in the second state, the delivery channel is in a closed state.

According to the delivery device, during interventional surgery, the delivery channel is opened or closed by moving the sheath connector in the hemostasis valve in the axial direction without the need for disassembling and assembling the hemostasis valve with the delivery sheath or the loader, thereby reducing surgical steps and shortening the operation time. Furthermore, the risk of leakage caused by untight fit between the delivery sheath and the hemostasis valve due to improper on-site manual assembly is avoided. Additionally, the adjusting nut rotatably surrounds the outside of the sheath connector, which cannot drive the sheath connector to move in a circumferential direction, but can only drive the sheath connector to move in the axial direction. In this way, the sheath connector is prevented from rotating in the circumferential direction when the position of the sheath connector is adjusted by the adjusting nut, thereby avoiding the detachment of other devices or instruments connected to the sheath connector, which may lead to surgical failure.

Further, a proximal end of the adjusting nut is rotatably connected to the sheath connector, the adjusting nut surrounds the valve seat, and the adjusting nut moves in the axial direction when rotated and accordingly drives the sheath connector to move in the axial direction.

Further, the elastic sealing member is provided with a slit extending through a proximal end and a distal end of the elastic sealing member; when the elastic sealing member is in the first state, the sheath connector is arranged in the slit in a penetrating manner, and the distal end of the sheath connector extends out of a distal end of the slit; and when the elastic sealing member in the second state, the proximal end of the sheath connector is positioned outside the slit, and the slit is closed to close the delivery channel.

Further, the delivery device includes a dilator, which includes a connecting member and a through-tube connected to the connecting member. The connecting member is detachably connected to the sheath connector, the through-tube is positioned inside the sheath connector, and a distal end of the through-tube extends out of the distal end of the sheath connector.

Further, the delivery device includes: an annular fixing member arranged in the valve seat. The annular fixing member is positioned close to the proximal end of the elastic sealing member and presses the elastic sealing member against the interior of the valve seat.

Further, the elastic sealing member protrudes from the proximal end toward the distal end; and/or a proximal opening of the slit is smaller than a distal opening of the slit.

Further, the elastic sealing member is of an annular structure and has a first opening extending through the proximal end and the distal end of the elastic sealing member. When the elastic sealing member is in the first state, the first opening communicates with the sheath connector; and when the elastic sealing member is in the second state, at least a portion of an inner wall of the first opening deforms toward the axis of the elastic sealing member to close the first opening so as to close the delivery channel.

Further, the delivery device includes a silicone tube. A proximal end of the silicone tube is connected to the distal end of the sheath connector, and the silicone tube is arranged in the first opening.

Further, the proximal opening of the silicone tube and the distal opening of the sheath connector have the same size and are joined with each other.

Further, the delivery device includes an auxiliary gasket arranged between the elastic sealing member and the sheath connector. The auxiliary gasket is a self-lubricating component.

Further, the distal end of the sheath connector is provided with a flared opening structure. An inner wall of the flared opening structure has a guiding inclined surface, and the guiding inclined surface is configured to press an outer peripheral surface of the elastic sealing member to compress the elastic sealing member in a radial direction.

Further, the valve seat is provided with a first guiding structure extending in the axial direction, and the sheath connector is provided with a second guiding structure that is in sliding fit with the first guiding structure to restrict the sheath connector from rotating in the circumferential direction.

Further, the delivery device includes a handle assembly. The proximal end of the hemostasis valve is inserted into the handle assembly.

According to a technical solution according to a second aspect of the present invention, a delivery system is proposed. The delivery system includes the delivery device according to the first aspect of the present invention and a loader that are connected to each other.

Further, the loader includes a connecting element and a loading sheath connected to the connecting element. The connecting element is detachably connected to a distal end of the sheath connector, and the loading sheath is communicated with the sheath connector.

### Brief Description of the Drawings

By reading the detailed description of the preferred embodiments below, various other advantages and benefits will become apparent to those skilled in the art. The drawings are provided only for the purpose of illustrating the preferred embodiments and are not intended to limit the present invention. Furthermore, the same numeral signs are used to denote the same components throughout the drawings. In the drawings:
FIG. 1 is a schematic structural diagram of a delivery device according to an embodiment of the present invention.
FIG. 2 is a schematic diagram of an exploded view of components of a delivery system according to Embodiment I of the present invention.
FIG. 3 is a schematic cross-sectional diagram of a partial structure of a delivery system according to Embodiment I of the present invention.
FIG. 4 is a schematic diagram of a partial structure of a delivery system according to an embodiment of the present invention.
FIG. 5 is a schematic cross-sectional diagram of a partial structure of a delivery system according to Embodiment II of the present invention.
FIG. 6 is a schematic diagram of an exploded view of components of a delivery system according to Embodiment II of the present invention.
FIG. 7 is a schematic structural diagram of an elastic sealing member and an annular fixing member according to Embodiment II of the present invention.
FIG. 8 is a schematic structural diagram of an elastic sealing member according to Embodiment II of the present invention.
FIG. 9 is a schematic structural diagram of a valve seat according to Embodiment II of the present invention.
FIG. 10 is a schematic structural diagram of an elastic sealing member according to an exemplary embodiment of Embodiment II of the present invention.
FIG. 11 is a schematic structural diagram of an elastic sealing member from another perspective according to an exemplary embodiment of Embodiment II of the present invention.
FIG. 12 is a schematic diagram of a cross-sectional structure of an elastic sealing member according to another embodiment of the present invention.
FIG. 13 is a schematic diagram of an exploded view of components of a delivery system according to an exemplary embodiment of Embodiment **III** of the present invention.
FIG. 14 is a schematic diagram of a cross-sectional structure of a delivery system according to an exemplary embodiment of Embodiment III of the present invention.
FIG. 15 is a schematic structural diagram of an elastic sealing member according to an exemplary embodiment of Embodiment III of the present invention.
FIG. 16 is a schematic diagram of an exploded view of components of a delivery system according to an exemplary embodiment of Embodiment III of the present invention.
FIG. 17 is a schematic structural diagram of an elastic sealing member and a sheath connector according to an exemplary embodiment of Embodiment III of the present invention.
FIG. 18 is a schematic diagram of a cross-sectional structure of a delivery system according to an exemplary embodiment of Embodiment III of the present invention.
FIG. 19 is a schematic diagram of an exploded view of components of a delivery system according to an exemplary embodiment of Embodiment III of the present invention.
FIG. 20 is a schematic diagram of a cross-sectional structure of a delivery system according to an exemplary embodiment of Embodiment III of the present invention.
FIG. 21 is a schematic structural diagram of a sheath connector and a loader according to Embodiment IV of the present invention.
FIG. 22 is a schematic structural diagram of a sheath connector and a loader according to Embodiment V of the present invention.
FIG. 23 is a schematic structural diagram of a delivery system according to Embodiment VI of the present invention.

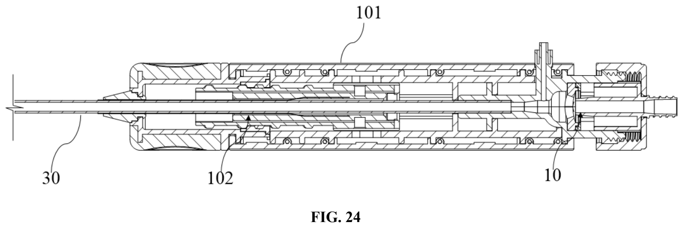

FIG. 24 is a schematic diagram of a cross-sectional structure of a delivery system according to Embodiment VI of the present invention.

### Detailed Description of the Invention

The exemplary embodiments of the present invention will be described in more detail below with reference to the accompanying drawings. Although the accompanying drawings show the exemplary embodiments of the present invention, it should be understood that the present invention can be implemented in various forms, and should not be limited to the embodiments stated herein. Rather, these embodiments are provided for understanding the present invention more thoroughly, and can completely transfer the scope of the present invention to those skilled in the art.

It should be understood that the terms used herein are only for the purpose of describing specific exemplary embodiments and are not intended to be restrictive. Unless otherwise explicitly stated in the context, the singular forms such as "a/an", "one", and "the" used herein can also indicate a plural form. The terms such as "comprise", "include", "contain", and "have" are inclusive and specify the presence of stated features, steps, operations, elements, and/or components but do not exclude the presence or addition of one or more other features, steps, operations, elements, components, and/or combinations thereof. The method steps, processes, and operations described herein should not be construed as requiring execution in the specific sequence described or stated unless explicitly stated otherwise. It should be also understood that additional or alternative steps may be used.

Although the terms first, second, third, etc. may be used herein to describe multiple elements, components, areas, layers, and/or sections, these elements, components, areas, layers, and/or sections should not be limited by these terms. These terms can only be used to distinguish one element, component, area, layer, or section from another element, component, area, layer, or section. Unless explicitly stated otherwise, terms like "first" and "second" and other numerical terms do not imply a particular sequence or order. Thus, a first element, component, region, layer, or section discussed below may alternatively be referred to as a second element, component, region, layer, or section without departing from the teachings of the exemplary embodiments.

For ease of description, spatial relative relationship terms can be used herein to describe a relationship between an element or feature and another element or feature shown in the figure, such as "internal", "external", "inner side", "outer side", "beneath", "below", "on", and "above". These spatial relative relationship terms mean including different orientations of a device in use or operation, in addition to the orientations depicted in the figures. For example, if the device in the figure is flipped, then the elements described as "beneath other elements or features" or "below other elements or features" will subsequently be oriented as "on other elements or features" or "above other elements or features". Therefore, the example term "below..." can include both above and below orientations. The device can be oriented in other ways (rotated 90 degrees or in other directions) and the spatial relative relationship descriptors used in the text are interpreted accordingly.

It should be noted that "distal end" and "proximal end" are used as directional words, which are commonly used terms in the field of interventional medical devices. "Distal end" refers to an end away from an operator during surgery, and "proximal end" refers to an end close to the operator during surgery. Axial direction refers to a direction parallel to a line connecting a distal center and a proximal center of the medical device; and radial direction refers to a direction perpendicular to the above-mentioned axial direction.

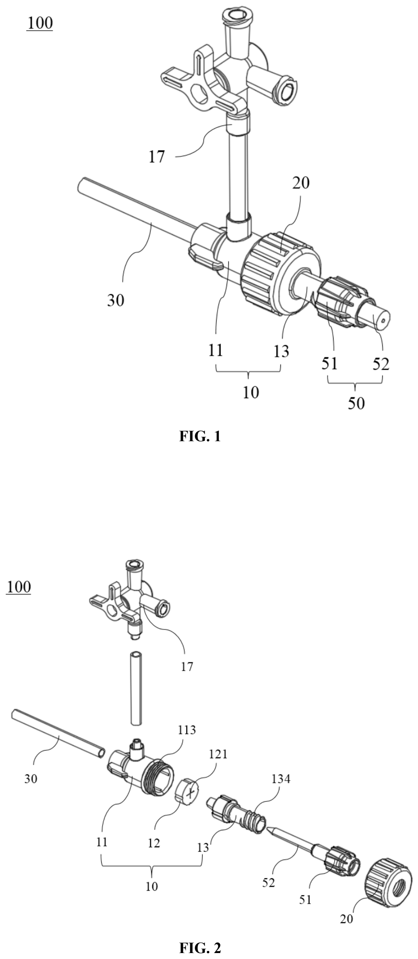

As shown in FIG. 1 and FIG. 2, according to an embodiment of the present invention, a delivery device 100 is proposed.

The delivery device 100 includes a hemostasis valve 10 and a delivery sheath 30. The hemostasis valve 10 includes a valve seat 11, an elastic sealing member 12, and a sheath connector 13. The elastic sealing member 12 is arranged in the valve seat 11. The sheath connector 13 is generally in a tubular shape and a distal end of the sheath connector 13 extends into the valve seat 11 and moves in the valve seat 11 in an axial direction. The valve seat 11, the elastic sealing member 12, and the sheath connector 13 are sequentially arranged from a distal end to a proximal end. The valve seat 11, the elastic sealing member 12, and the sheath connector 13 together define a delivery channel extending in the axial direction. That is, the delivery channel includes at least three sequentially connected segments, and the segments of the delivery channel are positioned in the valve seat 11, the elastic sealing member 12, and the sheath connector 13 respectively. The two ends of the delivery channel are communicated with the delivery sheath 30 and a proximal end of the sheath connector 13 respectively.

According to the delivery device 100 proposed in this embodiment, the sheath connector 13 presses the elastic sealing member 12 within a moving path to enable the elastic sealing member to switch between a first state and a second state. When the elastic sealing member is in the first state, the delivery channel is in an open state to allow the channel to remain unobstructed, so that a medical device or drug can pass through the delivery channel and the delivery sheath 30 and be delivered to a treatment site. When the elastic sealing member is in the second state, the delivery channel is in a closed state to prevent blood leakage. It can be understood that during interventional surgery, the delivery channel is opened or closed by moving the sheath connector 13 in the hemostasis valve 10 in the axial direction without the need for assembling or disassembling the hemostasis valve 10 with the delivery sheath 30 and a medical device or loader, thereby reducing surgical steps and shortening the operation time. Furthermore, the risk of leakage caused by untight fit between the delivery sheath 30 and the hemostasis valve 10 due to improper on-site manual assembly is avoided.

The technical solutions of the present invention will now be further described with reference to the specific embodiments.

### Embodiment I

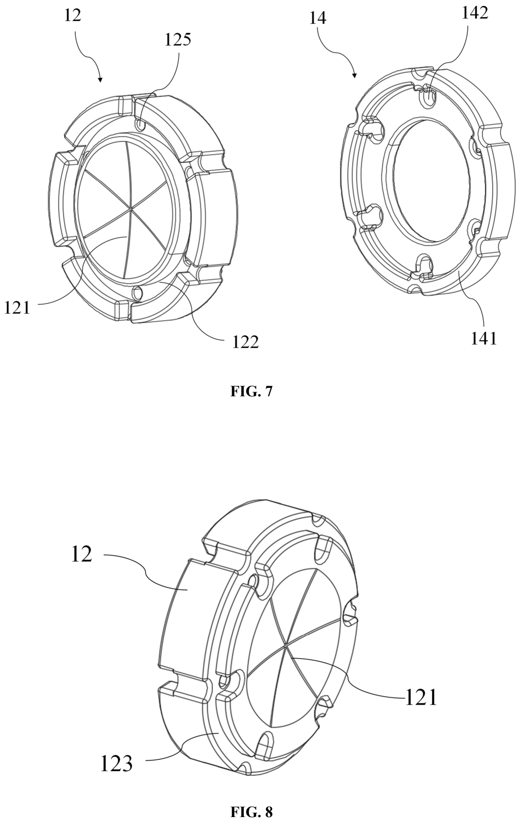

In this embodiment, as shown in FIG. 1, FIG. 2, and FIG. 3, the delivery device 100 further includes an adjusting nut 20. Specifically, the valve seat 11 includes a first channel 111 and an accommodating cavity 112, which are communicated with each other. The first channel 111 is closer to the distal end compared to the accommodating cavity 112. The diameter of the accommodating cavity 112 is greater than that of the first channel 111. A delivery opening 1111 is formed at a proximal end of the first channel 111 and on a distal wall surface of the accommodating cavity 112 (see FIG. 8). The distal end of the first channel 111 communicates with the delivery sheath 30. The elastic sealing member 12 is arranged in the accommodating cavity 112 and abuts against the distal wall surface of the accommodating cavity 112. The elastic sealing member 12 blocks the delivery opening 1111 and is configured to open or close the delivery opening 1111 as needed. The sheath connector 13 is arranged on a side away from the first channel 111 of the elastic sealing member 12. The distal end of the sheath connector 13 is movably arranged in the accommodating cavity 112 in the axial direction and abuts against the elastic sealing member 12. The proximal end of the sheath connector 13 extends out of the accommodating cavity 112 and is connected to a medical device or loader. The first channel 111 constitutes a distal segment of the delivery channel, the elastic sealing member 12 constitutes a middle segment of the delivery channel, and an inner lumen of the sheath connector 13 constitutes a proximal segment of the delivery channel.

In this embodiment, the elastic sealing member 12 is of a cylindrical structure, and the contour of an outer peripheral surface of the elastic sealing member 12 is matched with the contour of an inner peripheral surface of the accommodating cavity 112, thereby ensuring a tight fit between the contour of the outer peripheral surface of the elastic sealing member 12 and the contour of the inner peripheral surface of the accommodating cavity 112. The elastic sealing member 12 is provided with a cross slit 121 that extends through a proximal end and a distal end of the elastic sealing member 12. The cross slit 121 may be a cross-shaped slit formed by intersecting two linear slits, an asterisk-shaped slit formed by a plurality of linear slits, or a single linear slit. In the absence of external force, the cross slit 121 naturally extends and remains closed, thereby blocking the delivery opening 1111. The distal end of the sheath connector 13 is configured as a conical structure to facilitate insertion into and pressing of the cross slit 121. When the distal end of the sheath connector 13 is positioned close to the proximal end of the elastic sealing member 12, i.e., when the proximal end of the sheath connector 13 is positioned outside the cross slit 121, the elastic sealing member 12 is in the second state, and the cross slit 121 remains closed to block the delivery opening 1111, thereby blocking the delivery channel. When the sheath connector 13 moves toward the distal end, the distal end of the sheath connector 13 is inserted into the cross slit 121, presses the cross slit 121, and causes the elastic sealing member 12 at the cross slit to deform toward a side far away from the axis. When the distal end of the sheath connector 13 passes through and extends out of the distal end of the cross slit, the inner lumen of the sheath connector 13 is directly communicated with the first channel 111, thereby enabling the entire delivery channel to be in the open state.

In this embodiment, the elastic sealing member 12 is a silicone component made from a silicone material. Under the external force, the silicone component exhibits high elasticity and deformation capacity, as well as excellent elastic recovery performance, which enables the elastic sealing member 12 to switch between the first state and the second state. Alternatively, the elastic sealing member 12 may be made from materials such as latex or polyurethane, which are chemically stable and exhibit elastic properties.

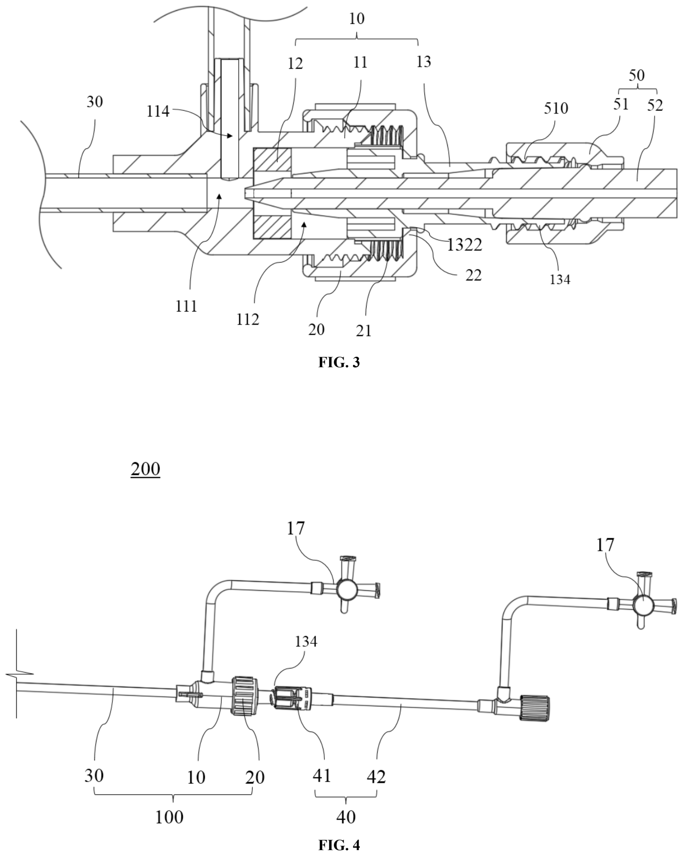

As shown in FIG. 2 and FIG. 3, the adjusting nut 20 rotatably surrounds the outside of the sheath connector 13. The adjusting nut 20 drives the sheath connector 13 to move in the axial direction. In this embodiment, the proximal end of the adjusting nut 20 is rotatably connected to the sheath connector 13, and the adjusting nut 20 surrounds the valve seat 11. The adjusting nut 20 moves in the axial direction when rotated and accordingly drives the sheath connector 13 to move in the axial direction. Specifically, a first external thread 113 is arranged on a side close to the proximal end of an outer peripheral surface of the valve seat 11. A first internal thread 21 that is aligned with the first external thread 113 is arranged on an inner wall of the adjusting nut 20 in a circumferential direction and allows the adjusting nut 20 to achieve movable connection with the valve seat 11 in a screw-in manner. A first annular protrusion 22 is arranged on a side close to the proximal end of an inner wall surface of the adjusting nut 20 in the circumferential direction. A first annular groove 1322 is formed on an outer peripheral surface of the sheath connector 13 in the circumferential direction. The first annular protrusion 22 is rotatably arranged in the first annular groove 1322. When the adjusting nut 20 is rotated to move relative to the valve seat 11 in the axial direction, the fit between sidewalls of the first annular protrusion 22 and the first annular groove 1322 drives the sheath connector 13 to move in the axial direction. That is, the delivery channel is opened or closed by rotating the adjusting nut 20 in a clockwise or anticlockwise direction, without driving the sheath connector 13 to rotate in the circumferential direction. Additionally, the adjusting nut 20 rotatably surrounds the outside of the sheath connector 13, which cannot drive the sheath connector 13 to rotate in the circumferential direction, but can only drive the sheath connector 13 to move in the axial direction. In this way, the sheath connector 13 is prevented from rotating in the circumferential direction when a position of the sheath connector 13 is adjusted by the adjusting nut 20, thereby avoiding the detachment of other devices or instruments connected to the sheath connector 13, which may lead to surgical failure.

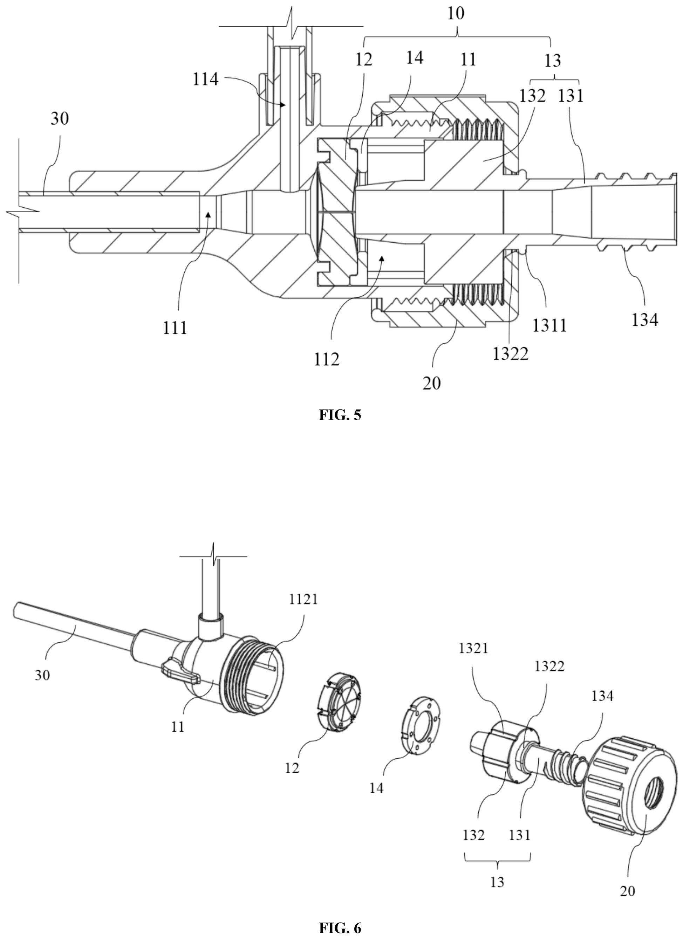

In this embodiment, as shown in FIG. 5, the sheath connector 13 includes a tubular body portion 131 and a sliding fit portion 132 arranged on an outer peripheral surface of the tubular body portion 131. An outer peripheral surface of the sliding fit portion 132 is in a sliding fit with a circumferential inner wall of the accommodating cavity 112. By the fit between the sliding fit portion 132 and the accommodating cavity 112, the sheath connector 13 moves in the axial direction. A protruding ring 1311 is arranged on the outer peripheral surface of the tubular body portion 131. The protruding ring 1311 and the sliding fit portion 132 are arranged at intervals in the axial direction, the protruding ring 1311 is positioned close to the proximal end of the sliding fit portion 132, and the first annular groove 1322 is defined between the protruding ring 1311 and the sliding fit portion 132.

It can be understood that in other embodiments, the adjusting nut rotatably surrounds the outside of both the valve seat and the sheath connector. The inner wall of the adjusting nut is provided with the first internal thread in the circumferential direction, and the first annular protrusion is arranged on the side close to the distal end of the inner wall surface of the adjusting nut in the circumferential direction. The outer peripheral surface of the valve seat is provided with the first annular groove in the circumferential direction, and the first annular protrusion is rotatably arranged in the first annular groove. The outer peripheral surface of the sheath connector is provided with the first external thread that is in fit with the first internal thread. When the adjusting nut is rotated, the fit between the sidewalls of the first annular protrusion and the first annular groove keeps the adjusting nut stationary relative to the valve seat in the axial direction. Simultaneously, the fit between the first internal thread and the first external thread drives the sheath connector to move relative to the adjusting nut and the valve seat in the axial direction, thereby opening or closing the delivery channel. In this embodiment, a device for restricting movement of the sheath connector in the circumferential direction is further required, such as a first guiding structure 1121 and a second guiding structure 1321 shown in FIG. 6, which prevent circumferential movement of the sheath connector.

As shown in FIG. 6, the valve seat 11 may further be provided with a first guiding structure 1121 that extends in the axial direction. The sheath connector 13 is provided with a second guiding structure 1321 that is in sliding fit with the first guiding structure 1121. Under the guiding action of the first guiding structure 1121 and the second guiding structure 1321, the sheath connector 13 moves relative to the valve seat 11 in the axial direction, while the sheath connector 13 is prevented from rotating relative to the valve seat 11 in the circumferential direction. Therefore, the circumferential positions of the delivery sheath 30 and the loader 40 can be stably maintained, thereby further facilitating the operation of the delivery system.

It should be noted that in this embodiment, as shown in FIG. 6, the first guiding structure 1121 is arranged on the circumferential inner wall of the accommodating cavity 112. The first guiding structure 1121 includes a plurality of guiding protrusions and the length of the guiding protrusions extends in the axial direction. The plurality of guiding protrusions are arranged at intervals on the inner wall of the accommodating cavity 112 in the circumferential direction. The second guiding structure 1321 is arranged on the outer peripheral surface of the sheath connector 13. Specifically, the second guiding structure 1321 includes guiding grooves formed on the outer peripheral surface of the sliding fit portion 132. The length of guiding groove extends in the axial direction and the plurality of guiding grooves are arranged at intervals on the outer peripheral surface of the sliding fit portion 132 in the circumferential direction. Each guiding groove corresponds to one guiding protrusion. The guiding protrusions are slidingly arranged in the guiding grooves in the axial direction. The fit between the guiding protrusions and the guiding grooves prevents the sheath connector 13 from rotating relative to the valve seat 11 in the circumferential direction.

In other embodiments, the first guiding structure is implemented as guiding grooves, while the second guiding structure is implemented as guiding protrusions.

Further, in this embodiment, as shown in FIG. 2 and FIG. 3, the delivery device 100 further includes a dilator 50, which includes a connecting member 51 and a through-tube 52 that are connected to each other. Specifically, the connecting member 51 surrounds the outside of the through-tube 52 and rotates relative to the through-tube 52 in the circumferential direction. The through-tube 52 extends in the sheath connector 13 and the distal end of the through-tube 52 extends out of the distal end of the sheath connector 13. A second annular protrusion is arranged on an outer peripheral surface of the through-tube 52 in the circumferential direction. A boss structure that is in stop-fit with the second annular protrusion (not shown) is arranged on a side at the proximal end of the second annular protrusion of a circumferential inner wall of the connecting member 51. This configuration allows the connecting member 51 and the through-tube 52 to rotate relative to each other in the circumferential direction. Additionally, a third internal thread 510 that is in fit with the second external thread 134 is arranged on a side close to the distal end of the circumferential inner wall of the connecting member 51. When the connecting member 51 is rotated, the fit between the second external thread 134 and the third internal thread 510 enables the connecting member 51 to move relative to the sheath connector 13 in the axial direction. This axial movement can push the through-tube 52 to move toward the distal end relative to the sheath connector 13, and cause the distal end of the through-tube 52 to extend through and dilate the cross slit 121. During this process, the connecting member 51 does not drive the through-tube 52 to rotate in the circumferential direction. Specifically, the distal end of the through-tube 52 is configured as a conical structure to facilitate insertion of the through-tube 52 into the cross slit 121.

It should be emphasized that in the delivery system proposed by the present invention, the dilator is not an essential component. The function of the dilator is to assist the sheath connector in inserting into the cross slit. In some embodiments, the dilator is omitted, and the sheath connector directly extends through the cross slit.

It should be noted that after the proximal end of the sheath connector 13 is inserted into the cross slit 121, the dilator 50 is required to be removed from the sheath connector 13 before connecting the sheath connector 13 to other instruments.

In this embodiment, as shown in FIG. 1 and FIG. 3, the delivery system further includes a three-way valve 17. The valve seat 11 is provided with a second channel 114 that is communicated with the first channel 111. The three-way valve 17 is communicated with the second channel 114. In this way, during surgery, medication or other substances required for surgery can be injected into the first channel 111 and the delivery sheath 30, or air evacuation and other operations can be performed through the three-way valve 17.

As shown in FIG. 4, this embodiment further provides a delivery system 200. The delivery system 200 includes the delivery device 100 and a loader 40. The delivery device 100 is connected to the loader 40.

In this embodiment, the loader 40 includes a connecting element 41 and a loading sheath 42 connected to the connecting element 41. A second external thread 134 is arranged on the outer peripheral surface close to the proximal end of the sheath connector 13. The connecting element 41 surrounds the outside of the sheath connector 13, and a second internal thread (not shown) that is in fit with the second external thread 134 is arranged on an inner wall surface of the connecting element 41. The threaded fit between the second external thread 134 and the second internal thread allows for the detachable connection between the connecting element 41 and the sheath connector 13 and allows for communication between the loading sheath 42 and the sheath connector 13. In this way, under a pushing force of a delivery cable, a medical device accommodated in the loader 40 can pass through the sheath connector 13 and the first channel 111, and enter the delivery sheath 30.

### Embodiment II

The structures of the delivery sheath 30, the loader 40, and the sheath connector 13 in this embodiment are essentially the same as those in Embodiment I. The primary difference lies in the structures of the valve seat 11 and the elastic sealing member 12. The following describes the differences between Embodiment II and Embodiment I, while the similarities or identical features between Embodiment I and Embodiment II will not be repeated herein.

In this embodiment, as shown in FIG. 5 and FIG. 6, a delivery device 100 further includes an annular fixing member 14. The annular fixing member 14 is arranged in an accommodating cavity 112 and is fixedly connected to the valve seat 11. The annular fixing member 14 is positioned at a proximal end of the elastic sealing member 12 and presses the elastic sealing member 12 against a distal wall surface of the accommodating cavity 112. The primary function of the annular fixing member 14 is to secure the elastic sealing member 12 and press the elastic sealing member against the distal wall surface of the accommodating cavity 112, thereby enhancing the adherence effect of the elastic sealing member 12 to the wall, ensuring good closure of a delivery opening 1111, and preventing blood leakage. Further, the annular fixing member 14 may be made of a material with self-lubricating properties, such as polyamide (PA) or polyoxymethylene (POM), to reduce the force required for the sheath connector 13 to enter the elastic sealing member 12 and facilitate insertion of the sheath connector 13 into the elastic sealing member 12.

In an exemplary embodiment, as shown in FIG. 5 to FIG. 9, a plurality of clamping posts 1123 are arranged on the distal wall surface of the accommodating cavity 112. The clamping posts 1123 are in a columnar shape. Axes of the clamping posts 1123 extend in the axial direction. The plurality of clamping posts 1123 are arranged at intervals around the delivery opening 1111. The elastic sealing member 12 is provided with a plurality of through-holes 125 that extend through the proximal end and the distal end of the elastic sealing member 12. Each through-hole 125 corresponds to one clamping post 1123, thereby allowing the clamping posts 1123 to pass through the through-holes 125 and extend out of proximal ends of the through-holes 125. The annular fixing member 14 is provided with a plurality of clamping holes 142 and each clamping hole corresponds to one clamping post 1123. The clamping posts 1123 are inserted into the clamping holes 142, thereby achieving a fixed connection between the annular fixing member 14 and the valve seat 11. The clamping posts 1123 and the clamping holes 142 are in interference fit, thereby ensuring that the annular fixing member 14 is securely connected to the valve seat 11. The phenomenon that the elastic sealing member 12 may be loosened by the pressing of the sheath connector 13 is therefore avoided.

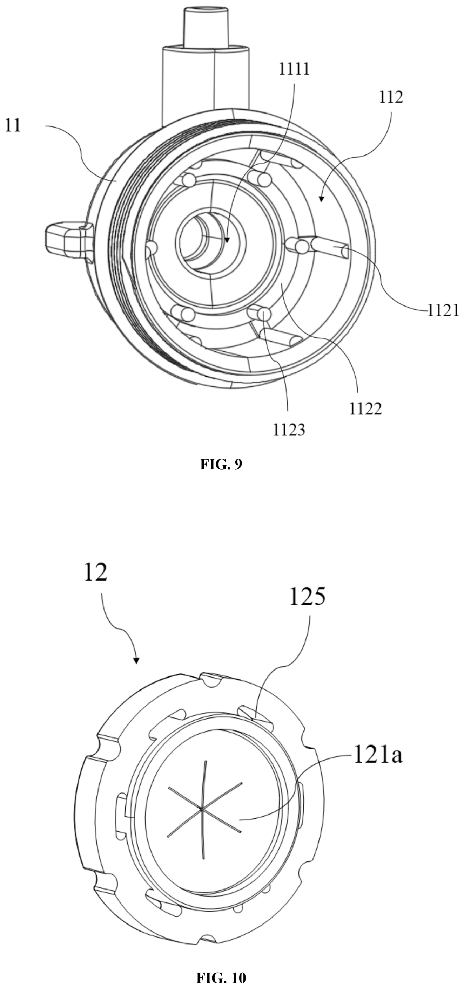

In this embodiment, as shown in FIG. 9, each clamping post 1123 corresponds to one guiding protrusion. Under the guiding action of guiding protrusions and guiding grooves, the clamping posts 1123 can accurately align with the through-holes 125, and the clamping posts 1123 can smoothly pass through the through-holes 125 during installation. It should also be noted that the clamping posts 1123 are arranged in the through-holes 125 in a penetrating manner, whereby the elastic sealing member 12 is prevented from rotating relative to the valve seat 11 in the circumferential direction and weakening of the sealing performance of the elastic sealing member 12 when the sheath connector 13 presses the elastic sealing member 12 is avoided.

In an exemplary embodiment, as shown in FIG. 5 to FIG. 9, a first annular convex rib 1122 is arranged on the distal wall surface of the accommodating cavity 112, and the plurality of clamping posts 1123 are all arranged on the first annular convex rib 1122. The first annular convex rib 1122 is arranged around the delivery opening 1111. A first annular fit groove 122 is arranged on a distal end surface of the elastic sealing member 12. The first annular fit groove 122 is arranged around a cross slit 121. The contour of the first annular convex rib 1122 is matched with that of the first annular fit groove 122. When the elastic sealing member 12 is pressed against the distal wall surface of the accommodating cavity 112, the first annular convex rib 1122 extends into the first annular fit groove 122. It should be noted that by arranging the first annular convex rib 1122 and the first annular fit groove 122, a plurality of continuous sealing surfaces are formed between the distal end surface of the elastic sealing member 12 and the distal wall surface of the accommodating cavity 112. The sealing surfaces are not coplanar, whereby when the sheath connector 13 is arranged in the cross slit 121 in a penetrating manner and presses the elastic sealing member 12 to cause deformation of the elastic sealing member 12, at least one sealing surface remains in a sealing state to prevent blood leakage. Consequently, the plurality of non-coplanar sealing surfaces improve the sealing performance between the elastic sealing member 12 and the distal end surface of the accommodating cavity 112. Additionally, when the elastic sealing member 12 is pressed against the distal wall surface of the accommodating cavity 112, the first annular convex rib 1122 is arranged outside and surrounds the cross slit 121. The first annular convex rib 1122 is made of a rigid material. When the cross slit 121 is pressed to cause the elastic sealing member 12 at the cross slit 121 to deform in a direction away from the axis, an inner wall of the first annular convex rib 1122 provides a support force toward the axis, thereby reducing the overall deformation of the elastic sealing member 12, ensuring that at least part of the sealing surfaces maintains the sealing state, and preventing blood leakage caused by inadequate sealing due to deformation of the elastic sealing member 12.

Further, as shown in FIG. 7 and FIG. 8, a distal end surface of the annular fixing member 14 is provided with a second annular convex rib 141. A proximal end surface of the elastic sealing member 12 is provided with a second annular fit groove 123. The second annular fit groove 123 is arranged outside and surrounds the cross slit 121. The contour of the second annular convex rib 141 is matched with the contour of the second annular fit groove 123. When the annular fixing member 14 is pressed against the proximal end surface of the elastic sealing member 12, the second annular convex rib 141 extends into the second annular fit groove 123. It should be noted that by arranging the second annular convex rib 141 and the second annular fit groove 123, when the annular fixing member 14 is pressed against the proximal end surface of the elastic sealing member 12, the second annular convex rib 141 is arranged outside and surrounds the cross slit 121 and provides a certain support; when the cross slit 121 is pressed to cause the elastic sealing member 12 at the cross slit 121 to deform in the direction away from the axis, an inner wall of the second annular convex rib 141 further provides a support toward the axial direction, thereby reducing the overall deformation of the elastic sealing member 12, ensuring that at least part of the sealing surface maintains the sealing state, and preventing blood leakage caused by inadequate sealing due to deformation of the elastic sealing member 12.

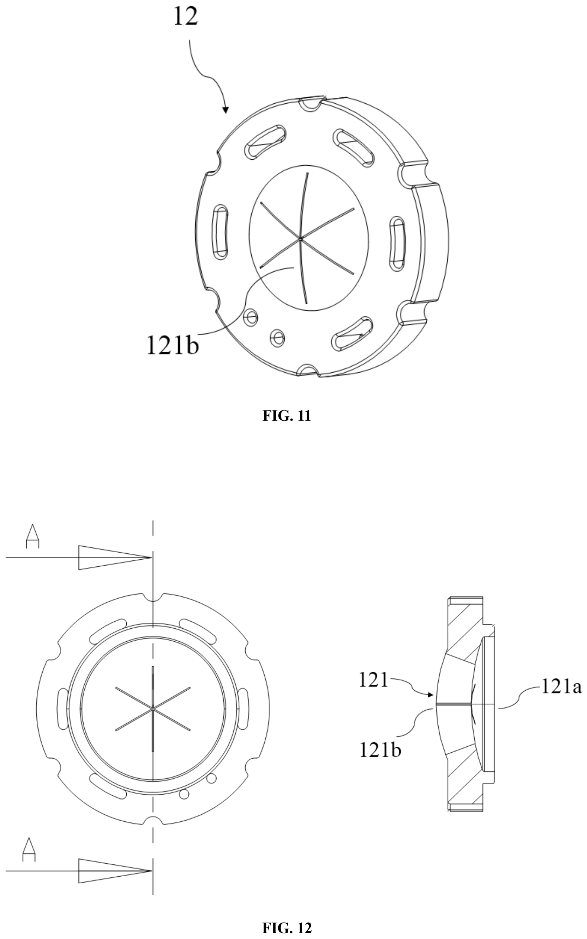

As shown in FIG. 5, in this embodiment, the proximal end and the distal end of the elastic sealing member 12 are respectively recessed toward the center of the elastic sealing member 12. Additionally, as shown in FIG. 7 and FIG. 8, a size of the cross slit 121 remains constant from a proximal end to a distal end. In another embodiment, as shown in FIG. 10 to FIG. 12, the proximal end at the position of the cross slit 121 of the elastic sealing member 12 protrudes toward the distal end. This design facilitates the insertion of another device, such as the loader, from the proximal end to the distal end. Simultaneously, a device already inserted is more easily secured at the position of the cross slit 121 of the elastic sealing member 12 and is less likely to be withdrawn from the distal end to the proximal end. Therefore, operational stability and safety can be improved. Furthermore, a proximal opening 121a of the cross slit 121 is smaller than a distal opening 121b, thereby improving sealing performance.

### Embodiment III

The structures of the delivery sheath 30 and the loader 40 in this embodiment are essentially the same as those in Embodiment I, and the main differences lie in the structures of the elastic sealing member 12 and the sheath connector 13 in a hemostasis valve 10. The following describes the differences between Embodiment III and Embodiment I or Embodiment II, while the similarities or identical features between Embodiment III and Embodiment I or Embodiment II will not be repeated herein.

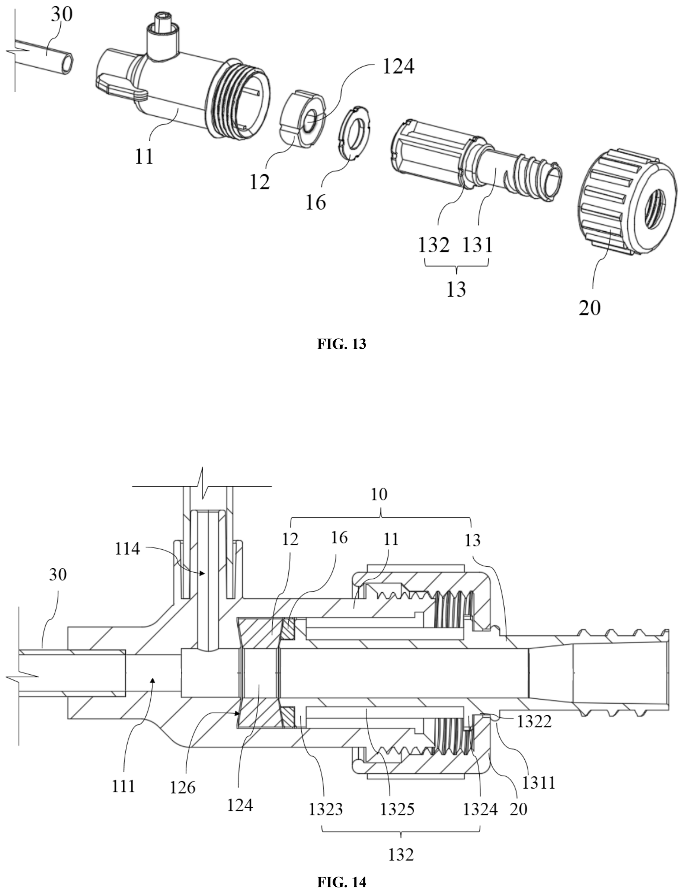

In this embodiment, as shown in FIG. 13 and FIG. 14, the elastic sealing member 12 is of an annular structure and is provided with a first opening 124 that extends through the proximal end and distal end of the elastic sealing member 12. Specifically, the elastic sealing member 12 is positioned between a first channel 111 and the sheath connector 13. The first channel 111, the first opening 124, and an inner lumen of the sheath connector 13 are sequentially communicated to form a delivery channel. That is, the first channel 111 constitutes a distal segment of the delivery channel, the first opening 124 constitutes a middle segment of the delivery channel, and the inner lumen of the sheath connector 13 constitutes a proximal segment of the delivery channel.

The distal end of the sheath connector 13 is configured to abut against a proximal end surface of the elastic sealing member 12. This configuration enables the sheath connector 13 to press the elastic sealing member 12 when moving in an axial direction, causing deformation of the elastic sealing member 12. Specifically, when the elastic sealing member 12 is in a first state, the first channel 111 is communicated with the sheath connector 13 through the first opening 124. When the elastic sealing member 12 in a second state, at least a portion of an inner wall of the first opening 124 deforms toward the axis of the elastic sealing member 12 to close the first opening 124 so as to close the delivery channel.

In an exemplary embodiment, as shown in FIG. 13 and FIG. 14, to facilitate pressing of the sheath connector 13 against the elastic sealing member 12 and to ensure a smoother deformation of the elastic sealing member 12, the hemostasis valve 10 further includes an auxiliary gasket 16. The auxiliary gasket 16 is arranged between the elastic sealing member 12 and the sheath connector 13. The auxiliary gasket 16 is connected to the distal end of the sheath connector 13, and abuts against a proximal end of a gasket of the elastic sealing member. In this embodiment, a sliding fit portion 132 includes a first baffle portion 1323, a second baffle portion 1324, and at least one supporting rib 1325. The first baffle portion 1323 and the second baffle portion 1324 are arranged on an outer peripheral surface of a tubular body portion 131 in a circumferential direction and are arranged at intervals in an axial direction. The first baffle portion 1323 is closer to the distal end compared to the second baffle portion 1324. The supporting rib 1325 extends in the axial direction and is respectively connected to the first baffle portion 1323 and the second baffle portion 1324. The auxiliary gasket 16 surrounds the outside of the tubular body portion 131 and is positioned between the elastic sealing member 12 and the first baffle portion 1323. When the sheath connector 13 is pushed toward the distal end, the first baffle portion 1323 presses the elastic sealing member 12 via the auxiliary gasket 16 to cause the elastic sealing member 12 to deform. The auxiliary gasket 16 is made of a material with self-lubricating properties, such as PA or POM, to reduce friction between the elastic sealing member 12 and both the sheath connector 13 and an inner wall of the accommodating cavity 112 during deformation. Therefore, a smoother deformation of the elastic sealing member 12 is achieved. In this embodiment, a first annular groove 1322 is defined between a protruding ring 1311 and the second baffle portion 1324.

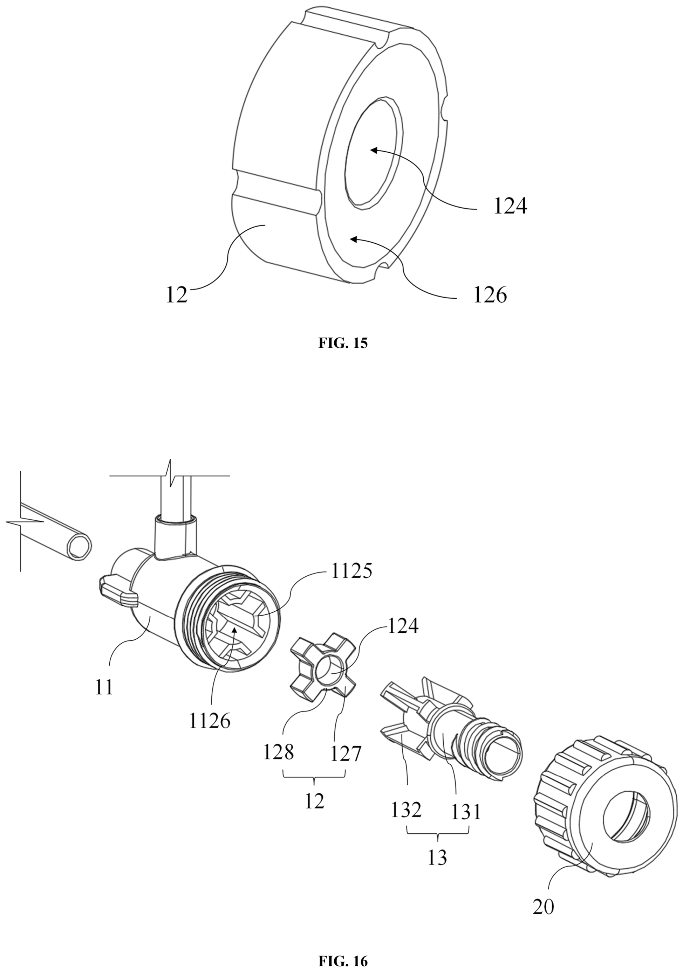

In this embodiment, as shown in FIG. 14 and FIG. 15, both proximal and distal end surfaces of the elastic sealing member 12 are configured as conical surfaces 126, and the axis of the conical surfaces 126 coincides with the axis of the first opening 124. A distal wall surface of the accommodating cavity 112 and a distal end surface of the auxiliary gasket 16 are both fitted to the conical surfaces 126. A guiding effect provided by the conical surfaces 126 facilitates axial alignment of the sheath connector 13 and the auxiliary gasket 16 to the elastic sealing member 12. In other embodiments, both the proximal and distal end surfaces of the elastic sealing member 12 are configured as flat surfaces.

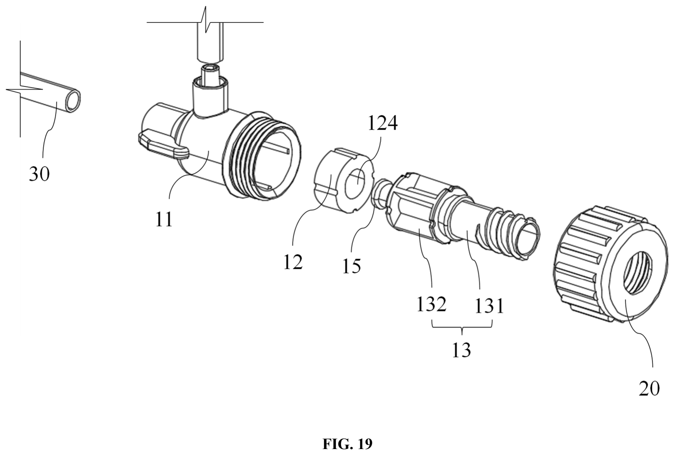

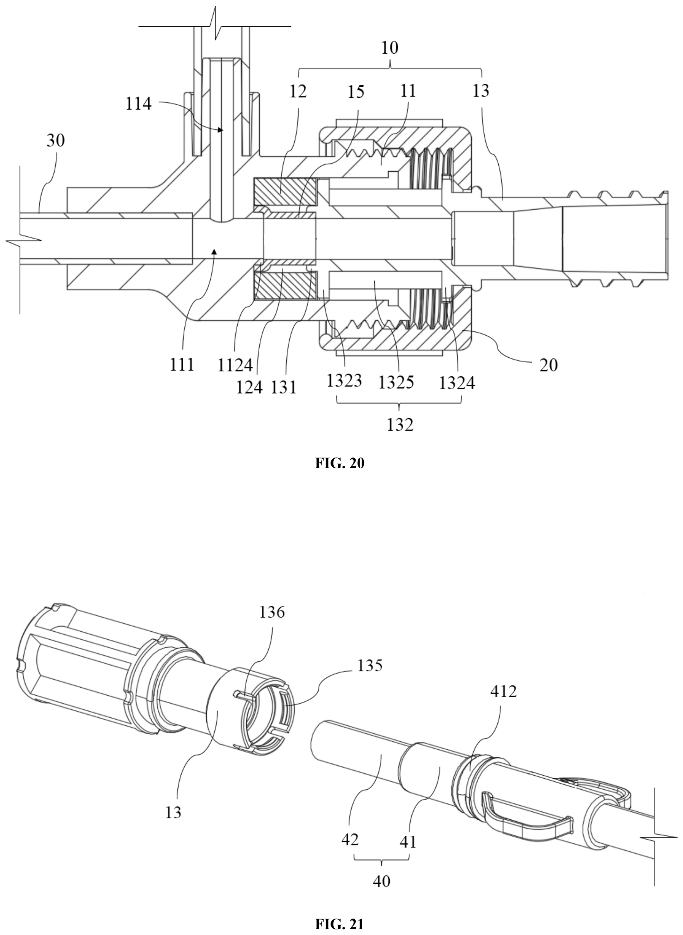

In another exemplary embodiment, as shown in FIG. 19 and FIG. 20, the hemostasis valve 10 further includes a silicone tube 15, which is positioned inside the first opening 124. A distal end of the silicone tube 15 abuts against the distal wall surface of the accommodating cavity 112, while a proximal end of the silicone tube 15 is connected to the distal end of the sheath connector 13. Further, a proximal opening of the silicone tube 15 and a distal opening of the sheath connector 13 have the same size and are joined with each other. It is understood that, prior to assembling the delivery device 100, the silicone tube 15 is assembled with the sheath connector 13, whereby a smooth connection between inner lumens of the silicone tube and the sheath connector is ensured to facilitate the delivery of a device or drug.

Specifically, the sliding fit portion 132 includes the first baffle portion 1323, the second baffle portion 1324, and the at least one supporting rib 1325. The first baffle portion 1323 and the second baffle portion 1324 are arranged on the outer peripheral surface of the tubular body portion 131 in the circumferential direction and are arranged at intervals in the axial direction. The first baffle portion 1323 is closer to the distal end compared to the second baffle portion 1324. The supporting rib 1325 extends in the axial direction and is respectively connected to the first baffle portion 1323 and the second baffle portion 1324. A distal end surface of the first baffle portion 1323 abuts against the proximal end surface of the elastic sealing member 12. A distal end of the tubular body portion 131 extends out of the distal end surface of the first baffle portion 1323 and is inserted into the first opening 124, and the outer peripheral surface of the distal end of the tubular body portion 131 abuts against an inner wall surface of the first opening 124. A limiting bulge loop 1124 is arranged on an edge surrounding the first opening 124 of the distal wall surface of the accommodating cavity 112. A gap is formed between the limiting bulge loop 1124 and the inner wall of the first opening 124 in the circumferential direction. The distal end of the silicone tube 15 is inserted into the gap between the limiting bulge loop 1124 and the inner wall of the first opening 124, and the proximal end of the silicone tube 15 is fixedly connected to the distal end of the tubular body portion 131. Specifically, an adjusting nut 20 drives the sheath connector 13 to move in the axial direction through threaded rotation. When the sheath connector 13 moves toward the distal end, the elastic sealing member 12 is pressed, and the elastic sealing member 12 in turn presses the silicone tube 15. Both the elastic sealing member 12 and the silicone tube 15 move toward the axis and deform, thereby achieving closure of the passage.

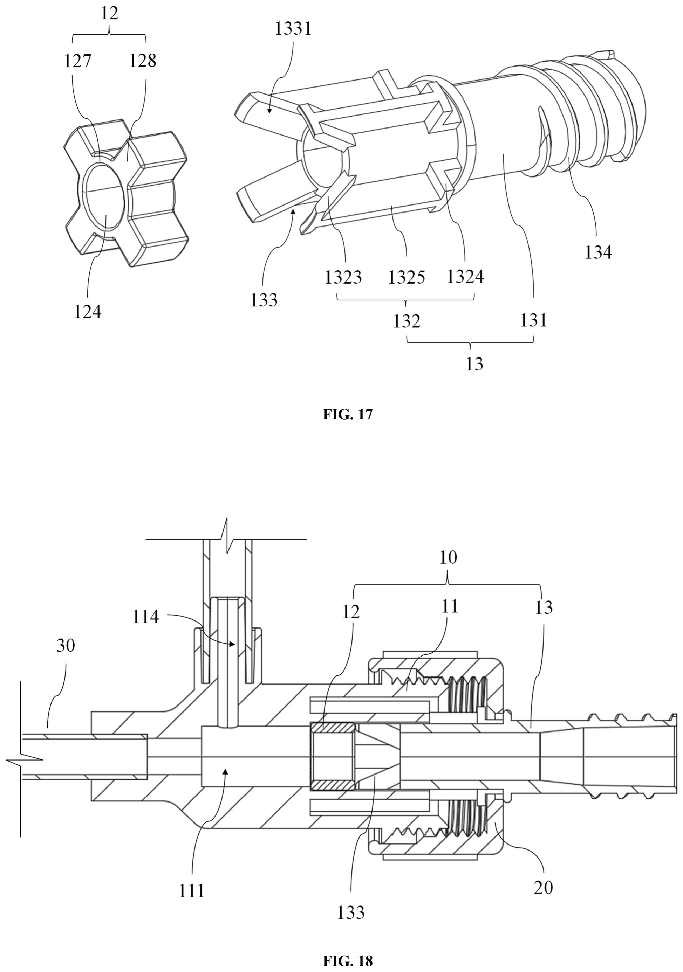

In another exemplary embodiment, as shown in FIG. 16, FIG. 17, and FIG. 18, the distal end of the sheath connector 13 is provided with a flared opening structure 133. An inner wall of the opening structure 133 has a guiding inclined surface 1331 that is configured to press an outer peripheral surface of the elastic sealing member 12 to induce radial compression of the elastic sealing member 12. The elastic sealing member 12 is pressed into the flared opening structure 133 to achieve radial compression, thereby closing the first opening 124.

Specifically, the elastic sealing member 12 includes a tubular gasket body 127 and a plurality of supporting portions 128 arranged around an outer peripheral surface of the gasket body 127. The plurality of supporting portions 128 are arranged at spaced intervals around the gasket body 127 sequentially in the circumferential direction, and the ends away from the gasket body 127 of the supporting portions 128 are fitted to the inner wall of the accommodating cavity 112. The sliding fit portion 132 includes a plurality of supporting ribs 1325, and the plurality of supporting ribs 1325 are arranged at intervals on the outer peripheral surface of the tubular body portion 131 in the circumferential direction. The length of the supporting ribs 1325 extends in the axial direction, a distal end of each supporting rib 1325 is provided with the first baffle portion 1323 and a proximal end of each supporting rib 1325 is provided with the second baffle portion 1324. The first baffle portions 1323 are arranged at a predetermined angle relative to the axis of the tubular body portion 131. Distal ends of the first baffle portions 1323 are far away from the axis of the tubular body portion 131 compared to proximal ends of the first baffle portions 1323, and thus the plurality of first baffle portions 1323 form the flared opening structure 133. A side face, facing the axis of the tubular body portion 131, of each first baffle portion 1323 is configured as the guiding inclined surface 1331 with a guiding function. Each first baffle portion 1323 corresponds to one supporting portion 128. When the sheath connector 13 moves toward the distal end and presses the elastic sealing member 12, the guiding inclined surfaces 1331 press the end surfaces of the sides away from the gasket body 127 of the supporting portions 128, thereby resulting in the radial compression of the elastic sealing member 12. The compression causes the elastic sealing member 12 to be forced into the flared opening structure 133, thereby closing the first opening 124 to close the delivery channel.

In this embodiment, the number of supporting portions 128 is same as that of supporting ribs 1325. All the supporting portions 128 are uniformly distributed around the gasket body 127 in the circumferential direction. All the supporting ribs 1325 are uniformly distributed around the tubular body portion 131 in the circumferential direction.

In this embodiment, as shown in FIG. 16, a plurality of limiting convex ribs 1125 are arranged in the accommodating cavity 112. The limiting convex ribs 1125 protrude from the distal wall surface of the accommodating cavity 112 toward the proximal end in the axial direction. The plurality of limiting convex ribs 1125 are arranged at intervals around the delivery opening 1111 in the circumferential direction. The limiting convex ribs 1125 and the circumferential inner wall of the accommodating cavity 112 form a guiding cavity 1126 for accommodating the elastic sealing member 12. The contour of an inner wall of the guiding cavity 1126 is matched with the contour of the outer peripheral surface of the elastic sealing member 12 to restrict the position of the elastic sealing member 12.

### Embodiment IV

The structures of the delivery sheath 30 and the hemostasis valve 10 in this embodiment are essentially the same as those in Embodiment **I,** Embodiment II or Embodiment III. The difference lies in the structures of the sheath connector 13 and the loader 40 in the hemostasis valve 10. The following describes the differences between Embodiment IV and Embodiment **I,** Embodiment II or Embodiment III, while the similarities or identical features between Embodiment I and Embodiment II or between Embodiment II and Embodiment **III** will not be repeated herein.

In this embodiment, as shown in FIG. 21, the loader 40 includes a connecting element 41 and a loading sheath 42 fixedly connected to the connecting element 41. A buckle 135 protruding from an inner wall is arranged on a side close to the proximal end of the inner wall of the sheath connector 13. A clamping slot 412 which is in clamping fit with the buckle 135 is formed on an outer peripheral surface of the connecting element 41. The connecting element 41 is inserted into the sheath connector 13, and achieves clamping through the fit of the buckle 135 and the clamping slot 412, thereby enabling quick assembly or disassembly of the delivery system.

Further, the proximal end of the sheath connector 13 is provided with at least one opening slot 136. An opening of the opening slot 136 is positioned at the proximal end of the sheath connector 13. When the connecting element 41 is inserted into the sheath connector 13, the arrangement of the opening slot 136 facilitates the expansion and deformation of the proximal end of the sheath connector 13, thereby allowing the connecting element 41 to be smoothly inserted into the sheath connector 13 for clamping. In this embodiment, the proximal end of the sheath connector 13 is provided with a plurality of opening slots 136, and the plurality of opening slots 136 are uniformly distributed around the axis of the sheath connector 13.

### Embodiment V

The structures of the delivery sheath 30 and the hemostasis valve 10 in this embodiment are essentially the same as those in Embodiment **I,** Embodiment II or Embodiment III. The difference lies in the structures of the sheath connector 13 and the loader 40 in the hemostasis valve 10. The following describes the differences between Embodiment V and Embodiment **I,** Embodiment **II,** or Embodiment III, while the similarities or identical features between Embodiment V and Embodiment **I,** Embodiment II or Embodiment **III** will not be repeated herein.

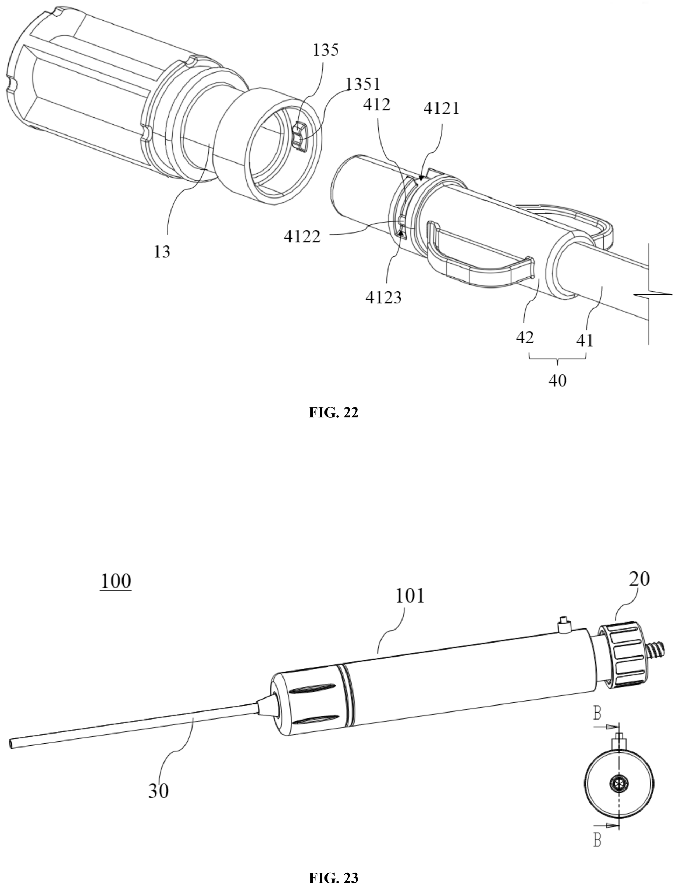

In this embodiment, as shown in FIG. 22, the loader 40 includes a connecting element 41 and a loading sheath 42 fixedly connected to the connecting element 41. A buckle 135 protruding from an inner wall is arranged on a side close to the proximal end of the inner wall of the sheath connector 13. A clamping slot 412 which is in clamping fit with the buckle 135 is formed on an outer peripheral surface of the connecting element 41. The connecting element 41 is inserted into the sheath connector 13, and achieves clamping through the fit between the buckle 135 and the clamping slot 412, thereby enabling quick assembly or disassembly of the delivery system.

Specifically, the length of the clamping slot 412 extends in a circumferential direction of the connecting element 41. The clamping slot 412 includes a first end and a second end in a longitudinal direction. A sidewall at the distal end of the first end is provided with an inlet 4121. When the connecting element 41 moves toward the distal end, the buckle 135 can enter the clamping slot 412 through the inlet 4121. The buckle 135 is provided with a buckle groove 1351, and a bump 4122 is formed on a bottom wall close to the second end of the clamping slot 412. After the buckle 135 enters the clamping slot 412 through the inlet 4121, the loader 40 is rotated in a circumferential direction to cause the buckle 135 to first abut against the bump 4122, and then the loader 40 is rotated continuously until the buckle groove 1351 is clamped with the bump 4122. At this point, one end of the buckle 135 abuts against a sidewall 4123 of the second end of the clamping slot 412, thereby achieving the clamping between the sheath connector 13 and the connecting element 41. Furthermore, during the overall axial movement of the loader 40, the fit between the buckle 135 and the distal or proximal sidewall of the clamping slot 412 prevents the loader 40 from detaching from the sheath connector 13.

### Embodiment VI

In the delivery device 100 described in Embodiments I to V, the delivery sheath 30 is directly connected to the hemostasis valve 10. In this embodiment, the delivery device 100 further includes a handle assembly 101. The proximal end of a hemostasis valve 10 is inserted into the handle assembly 101, and the proximal end of a delivery sheath 30 passes through the handle assembly 101 and enters the hemostasis valve 10. It should be understood that a deflection assembly 102 may be arranged in the handle assembly 101. The deflection assembly 102 has a structure well-known to those skilled in the art. For example, a sliding block is connected to the proximal end of a pull wire, the distal end of the pull wire is connected to the distal end of the delivery sheath 30, and the sliding block moves in an axial direction to drive the pull wire to move, thereby controlling the bending of the distal end of the delivery sheath 30. The specific structure is not elaborated here. Other structures may further be included within the handle assembly 101.

The above are only the specific embodiments of the present invention and not intended to limit the scope of protection of the present invention. Any variations or replacements apparent to those skilled in the art within the technical scope disclosed by the present invention shall fall within the scope of protection of the present invention. Therefore, the scope of protection of the present invention shall be subject to the scope of protection of the claims.

## Claims

1. A delivery device, comprising:
a delivery sheath;
a hemostasis valve, comprising a valve seat, an elastic sealing member, and a sheath connector, wherein the elastic sealing member is arranged in the valve seat, the elastic sealing member has a first state and a second state, a distal end of the sheath connector extends into the valve seat and moves in the valve seat in an axial direction, and the valve seat, the elastic sealing member, and the sheath connector together define a delivery channel that is communicated with the delivery sheath; and
an adjusting nut rotatably surrounding the outside of the sheath connector, wherein the adjusting nut drives the sheath connector to move in the axial direction,
wherein the sheath connector presses the elastic sealing member within a moving path to enable the elastic sealing member to switch between the first state and the second state, wherein when the elastic sealing member is in the first state, the delivery channel is in an open state, and when the elastic sealing member is in the second state, the delivery channel is in a closed state.

2. The delivery device according to claim 1, wherein a proximal end of the adjusting nut is rotatably connected to the sheath connector, the adjusting nut surrounds the valve seat, and the adjusting nut moves in the axial direction when rotated and accordingly drives the sheath connector to move in the axial direction.

3. The delivery device according to claim 1, wherein
the elastic sealing member is provided with a slit extending through a proximal end and a distal end of the elastic sealing member;
when the elastic sealing member is in the first state, the sheath connector is arranged in the slit in a penetrating manner, and the distal end of the sheath connector extends out of a distal end of the slit; and
when the elastic sealing member is in the second state, a proximal end of the sheath connector is positioned outside the slit, and the slit is closed to close the delivery channel.

4. The delivery device according to claim 3, further comprising:
a dilator, comprising a connecting member and a through-tube connected to the connecting member, wherein the connecting member is detachably connected to the sheath connector, the through-tube is positioned in the sheath connector, and a distal end of the through-tube extends out of the distal end of the sheath connector.

5. The delivery device according to claim 3, further comprising:
an annular fixing member arranged in the valve seat, wherein the annular fixing member is positioned at the proximal end of the elastic sealing member and presses the elastic sealing member against the interior of the valve seat.

6. The delivery device according to claim 3, wherein the elastic sealing member protrudes from the proximal end toward the distal end; and/or a proximal opening of the slit is smaller than a distal opening of the slit.

7. The delivery device according to claim 1, wherein
the elastic sealing member is of an annular structure and has a first opening extending through a proximal end and a distal end of the elastic sealing member;
when the elastic sealing member is in the first state, the first opening communicates with the sheath connector; and
when the elastic sealing member is in the second state, at least a portion of an inner wall of the first opening deforms toward the axis of the elastic sealing member to close the first opening so as to close the delivery channel.

8. The delivery device according to claim 7, further comprising:
a silicone tube, wherein a proximal end of the silicone tube is connected to the distal end of the sheath connector, and the silicone tube is arranged in the first opening.

9. The delivery device according to claim 8, wherein a proximal opening of the silicone tube and a distal opening of the sheath connector have the same size and are joined with each other.

10. The delivery device according to claim 7, further comprising:
an auxiliary gasket, arranged between the elastic sealing member and the sheath connector, wherein
the auxiliary gasket is a self-lubricating component.

11. The delivery device according to claim 7, wherein
the distal end of the sheath connector is provided with a flared opening structure, an inner wall of the opening structure has a guiding inclined surface, and the guiding inclined surface is configured to press an outer peripheral surface of the elastic sealing member to compress the elastic sealing member in a radial direction.

12. The delivery device according to claim 1, wherein
the valve seat is provided with a first guiding structure extending in the axial direction, and the sheath connector is provided with a second guiding structure that is in sliding fit with the first guiding structure to restrict the sheath connector from rotating in a circumferential direction.

13. The delivery device according to claim 1, further comprising a handle assembly, wherein a proximal end of the hemostasis valve is inserted into the handle assembly.

14. A delivery system, comprising the delivery device according to any one of claims 1 to 13 and a loader that are connected to each other.

15. The delivery system according to claim 14, wherein
the loader comprises a connecting element and a loading sheath connected to the connecting element, the connecting element is detachably connected to the distal end of the sheath connector, and the loading sheath communicates with the sheath connector.
